# EUROPEAN PATENT SPECIFICATION

(11) **EP 1 592 705 B1**
(45) Date of publication and mention of the grant of the patent: **05.05.2010**
(21) Application number: 04710463.3
(22) Date of filing: 12.02.2004
(51) Int. Cl.: C07K 14/38, C07K 14/385, C12N 15/31

(54) **DETECTION OF OCHRATOXIN A PRODUCING FUNGI**
AUFFINDEN VON PILZEN, DIE OCHRATOXIN A PRODUZIEREN
DETECTION DE CHAMPIGNONS PRODUISANT DE L'OCHRATOXINE A

(30) Priority: 12.02.2003 IE 20030095
(43) Date of publication of application: 09.11.2005
(73) Proprietor: University College Cork-National University of Ireland, Cork, Cork (IE)
(72) Inventor: Dobson, Alan, Cork (IE); O'Callaghan, John, Mallow, County Cork (IE)
(74) Representative: O'Brien, John Augustine
(86) International application number: PCT/IE2004/000020
(87) International publication number: WO 2004/072224

(56) References cited:
- DE-A- 19 628 959
- VARGA JANOS ET AL: "Ochratoxin production by Aspergillus species" APPLIED AND ENVIRONMENTAL MICROBIOLOGY, vol. 62, no. 12, 1996, pages 4461-4464, XP002292351 ISSN: 0099-2240
- DATABASE SWISSPROT 1 October 2002 (2002-10-01), WARD,T.J. ET AL.: "Trichothecene C-15 hydroxalase from strain NRRL28585 from Gibberella zeae (Fusarium graminearum)" XP002292353 Database accession no. Q8NJB3
- DATABASE SWISSPROT 1 November 1997 (1997-11-01), YU, J. ET AL.: "Averantin oxidoreductase (EC 1.14.-.-) (Cytochrome P450 60A1)." XP002292354 Database accession no. Q12732 -& YU JIUJIANG ET AL: "AvnA, a gene encoding a cytochrome P-450 monooxygenase, is involved in the conversion of averantin to averufin in aflatoxin biosynthesis in Aspergillus parasiticus" APPLIED AND ENVIRONMENTAL MICROBIOLOGY, vol. 63, no. 4, 1997, pages 1349-1356, XP002292352 ISSN: 0099-2240
- DATABASE EM_PAT [Online] EMBL; 20 September 2002 (2002-09-20), SEGAWA ET AL.: "Artificial primer" retrieved from EBI accession no. BD107450 Database accession no. BD107450

## Description

### Introduction

The invention relates to genes expressed by fungal cells producing the mycotoxin ochratoxin A (OTA) and a method for detecting ochratoxin A, particularly in food and feedstuffs.

### Background

Mycotoxins are a group of secondary metabolites which are produced by various filamentous fungi that can cause a toxic response termed a mycotoxicosis, if ingested by higher vertebrates and other animals.

Ochratoxin A (OTA) is a mycotoxin produced by *Aspergillus* and *Penicillium* species considered detrimental to human health and is classified as a possible human carcinogen. The recommended level for OTA in food for human consumption is 5µg/kg for raw grain, 3µg/kg for derived cereal products and 10µg/kg for dried vine fruits. The European Commission's Scientific Committee on Food has concluded that the intake of OTA should be reduced as far as possible to approximately 5ng per kilogram of body weight per day (Scientific Committee for food 1996).

Cereals normally correspond to 50 to 80% of average consumer intake. OTA is found mainly in wheat and barley (Kuiper-Goodman 1996; Pittet 1998). It is also found in coffee (Burdaspal and Legarda, 1998; Dietrich et al., 1995, Jorgenson, 1998), wine (Burdaspal and Legarda, 1999; Visconti et al., 1999; Zimmerli and Dick, 1996), beer (Visconti et al., 2000), pork (Jorgenson, 1998; Wolff et al, 2000) and grapes.

Conventional methods for fungal detection and identification in a sample involve plating, incubation and identification based on morphological characteristics. These methods are time-consuming, labour intensive and require experienced personnel that may be lacking in many laboratories to interpret the results.

There is therefore a clear need for a more accessible and improved method for the detection and identification of the presence of OTA producing fungi present at even very low levels in a sample.

### Statements of Invention

According to the invention there is provided use of a gene comprising the nucleotide sequence of SEQ ID No. 1 which is expressed by fungal cells producing ochratoxin A, in the detection and/or identification of ochratoxigenic fungi.

The invention also provides use of the gene which is upregulated during ochratoxin A biosynthesis, in the direction and/or identification of ochratoxigenic fungi.

The invention further provides use of a protein having an amino acid sequence derived from SEQ ID No 1 in the detection and/or identification of an ochratoxin A producing fungi. The use may be in the detection and/or identification of fungi that express ochratoxin A biosynthetic genes.

In one aspect the invention provides use of a nucleotide sequence of SEQ ID No. 1 in the detection and/or identification of an ochratoxigenic fungus.

The invention also provides use of a nucleotide sequence, of SEQ ID No. 1 in the construction of an atoxigenic strain of an ochratoxigenic fungus.

The ochratoxigenic fungus may be of the genus *Aspergillus*. The fungus may be of the species *carbonarius*, *niger, alliaceus* or *foetidus*.

Alternatively the ochratoxigenic fungus is of the genus *Penicillium*. The fungus may be of the species *verrucosum*.

The invention also provides an isolated nucleotide having SEQ ID No. 1.

The intention also provides Oligonucleotide primers derived from the isolated nucleotide,

| | | |
|---|---|---|
| AOH11-F: | 5' - agaacgggatgccaaaacagtgag - 3' | SEQ ID No. 18 |
| AQH11-R: | 5' - aagaatgcgegggatgggataacc - 3' | SEQ ID No. 19 |

In another aspect the invention provides an assay for the detection of ochratoxin A producing fungi comprising a nucleotide sequence of SEQ ID No. 1

The invention also provides an assay for the detection and identification of ochratoxin A producing genes comprising a nucleotide sequence of SEQ ID No. 1.
The invention further provides use of a nucleotide sequence of SEQ ID No. 1 to identify or isolate a nucleotide sequence, gene, peptide or polypeptide involved in ochratoxing A biosynthesis. The sequences may be used for so-called chromosome walling or gene library screening experiments to obtain other DNA sequences contiguous to the sequences involved in OTA production.

### Brief Description of the Drawings

The invention will be more clearly understood from the following descriptions thereof, given by way of example only, with reference to the accompanying drawings, in which:-
Figs. 1 (a) to (e) show the results of RT-PCR on the expression of cloned genes during OTA production by *Aspergillus ochraceus* HP99. Expression of Glyceraldehyde-3-phosphate dehydrogenase (G3PDH) (a) is used as a control. Samples were taken at 4, 5, 6, 7, 8 and 10 day time intervals;
Fig 2 shows the use of a PCR primer pair AOKS-RT2 to distinguish between genomic and cDNA. The 2B11 primer reactions are included for comparison.
Fig. 3 shows the results of thin layer chromatography (TLC) at each time interval;
Fig. 4 shows the results of a plate screening confirming the involvement of the *pks* gene in OTA biosynthesis. The blue fluorescent halo is indicated by an arrow.
Fig. 5 shows the results of TLC on atoxigenic strains of *A. ochraceus* (AO118 [lane 5]; AO107 [lane 4]; A053 [lane 3]) in comparison to ochratoxin A standards (lanes 1 and 7) and wild type *A ochraceus* extract (lanes 2 and 6). The position of OTA is indicated by the arrow.
Fig. 6 Shows RT-PCR analysis expression of the *pks* gene in *Aspergillus foetidus* ATCC 10254, using the 2B11 primer pair. During growth on (1) malt extract broth, (2) potato dextrose broth and (3) yeast extract glucose broth. Lanes 1, 2 and 3 are G3PDH controls. Lanes 1', 2' and 3' are the 2B11 primer reactions. +VE is positive control, -VE are negative controls.
Fig 7 Shows production of OTA by *A. foetidus* ATCC10254 mycelium from which the RNA for the above RT-PCR experiment was extracted.
Fig. 8 Shows RT-PCR measurement of expression of the *pks* gene by *Aspergillus niger* ATCC9029 using the GAP primer pair During growth on (1) malt extract broth, (2) potato dextrose broth and (3) yeast extract glucose broth. Lanes 1, 2 and 3 are G3PDH controls. Lanes 1', 2' and 3' are the 2B11 primer reactions. +VE is positive control, -VE are negative controls.

### Detailed Description

The disclosure provides isolated nucleotide sequences and genes expressed by fungal cells producing OTA and believed to be involved in the biosynthetic pathway for OTA biosynthesis.

The disclosure also provides detection methods based on these isolated nucleotide sequences to detect the presence of fungi producing OTA in a sample.

The detection methods provide simple and more rapid assays for the detection of ochratoxigenic fungi and have significant advantages over the detection methods currently available.

To date the biosynthetic pathway for OTA biosynthesis has not been characterised. However, the cloning and characterisation of the genes greatly increases the understanding of the biosynthetic pathway. The cloning and molecular characterisation of mycotoxin biosynthetic genes is vital in order to gain a fuller understanding of the organisation, regulation and expression of these genes.

In the invention suppression substractive hybridisation PCR (SSH-PCR) was used to clone a pool of cDNA sequences from *Aspergillus ochraceus* HP99 (University College Cork, Dept. of Microbiology, Culture Collection) that are expressed at higher levels during OTA biosynthesis. The cloned DNA sequences were then compared to sequences deposited in various databases in order to identify putative OTA biosynthetic genes.

A number of novel cloned DNA sequences were found to be expressed only by fungal cells producing OTA.

Polymerase chain reaction (PCR) based detection methods have the potential to detect and identify the present of mycotoxigenic fungi present at very low levels. (Geisen, 1998; Edwards *et al*., 2002).

Using the novel DNA sequence of the invention oligonucleotide primer pairs of the identified sequences were prepared. The oligonucleotide primer pairs form the basis of two nucleic acid based detection methods of the invention.

The detection methods of the invention use PCR and are extremely specific to the particular genus/species. They provide rapid detection using techniques which are easily learnt. The methods are based on the identification and isolation of the novel gene sequence of the invention which has been found to be specific to OTA.

In the first method a conventional PCR assay is used to detect fungi that have the capacity to produce OTA by indicating the presence of OTA an biosynthesis gene in fungal isolates. DNA from fungal isolates may be used as a template in PCR reactions. Oligonucleotide primers derived from the cloned gene sequence detect fungal isolates that are capable of producing OTA. Modem Real-time PCR based protocols can provide results in less than 40 minutes.

In the second method a reverse transcription (RT) - PCR based assay is used to identify fungi that are expressing the OTA biosynthetic gene and are therefore toxigenic. The RT-PCR assay detects the level of transcription (production of messenger RNA, mRNA) for a specific gene. The assay is performed in two stages (1) synthesis of cDNA from mRNA and (2) amplification of specific target sequences by PCR. The RT-PCR based assay provides highly specific detection of mRNA transcripts from the targeted genes. As RNA synthesis is the primary step in producing the enzymes necessary for any biosynthetic pathway the highly sensitive PCR based method demonstrates toxigenicity even when the amount of toxin produced is not detectable by conventional assays.

The isolation of the gene of the invention therefore has significant commercial benefit. The isolated DNA sequence may be used in a variety of assays for the detection of fungi producing ochratoxin A.

The PCR based detection system will detect the producing organism thereby allowing identification of potential problems from mycotoxin contamination prior to any significant amounts of toxin being produced.

A potential market for assays which can detect even the smallest amount of OTA producing fungi are the brewing, baking, wine making or animal feed industries as well as coffee manufacturers and cereal producers. The detection of OTA producing fungi or the presence of genes producing OTA at an early stage would ensure that the contaminated produce does not enter the food chain at source. Positive detection would lead to disposal of the contaminated produce and/or treatment to remove it.

The identification and isolation of the genes involved in the biosynthesis of OTA has valuable therapeutic potential. Mycotoxins are known to be involved in a number of disease states including cancer of the liver, damage of kidneys, weakening of the immune system, allergic reactions, ergotism and poisoning. Easy to use and rapid PCR based methods by which OTA producing fungi may be detected in source samples is therefore highly desirable.

The identification and characterisation of OTA producing genes is also important in determining the conditions under which the fungus produces the mycotoxin. The RT-PCR assay allows identification of the physiological parameters that promote ochratoxin production during storage such as water activity, temperature, pH etc. Storage conditions that are unfavourable to ochratoxin production can then be used to reduce or eliminate ochratoxin production in stored products. Information on the conditions that inhibit ochratoxin production are also essential to the use of HACCP to ensure mycotoxin-free food and animal feed.

The gene of the invention may also be used in the construction of atoxigenic strains of *A. ochraceus, A. carbonarius*, *A. alliaceus, A. niger, A. foetidus*, or *Penicillium verrucosum*. Atoxigenic strains are strains which are genetically engineered not to produce OTA. These strains may therefore be used in biocontrol strategies for the elimination of OTA production. In this way atoxigenic stains of fungi may be used without the risk of OTA being produced.

The gene of the invention may also be used to determine whether strains of *Aspergillus* strains which are used in industrial fermentations possess ochratoxin A biosynthetic genes i.e. the ability to produce the mycotoxin.

The gene of the invention may also be used to monitor whether OTA is being produced in industrial fermentations involving *Aspergillus* strains.

The invention will be more clearly understood from the following examples.

### Methodology

The method of SSH-PCR used is described in detail by Diatchenko *et al*. (1996) and a commercially available kit for the procedure is produced by Clontech laboratories (Palo Alto, CA, USA).

### (1) Development of permissive and restrictive growth media

The growth conditions under which *Aspergillus ochraceus* HP99 produced OTA (permissive) and did not produce OTA (restrictive) were identified. A number of different growth media were examined and it was observed that OTA was not produced when *A. ochraceus* was grown on Potato dextrose medium (PD) or malt extract medium (ME) either as liquid medium or solid medium. High levels of OTA production were observed if *A. ochraceus* was grown on yeast extract sucrose (YES) or Czapek-Dox medium containing yeast extract and casamino acids (MC).

### (2) SSH-PCR procedure

Total ribonucleic acid (RNA) was isolated from *A. ochraceus* HP99 mycelia grown on permissive (MC) and restrictive (PD) media. Double stranded complementary DNA (cDNA) was then synthesised from each RNA preparation using a SMART ™ cDNA synthesis kit (Clontech Laboratories, Palo Alto, CA, USA) and used to perform a suppression-subtractive hybridisation-PCR experiment (SSH-PCR) experiment. The success of the subtractive hybridisation in eliminating DNA sequences common to both samples was determined by quantitative PCR on the SSH-PCR product using primers to the constitutively expressed housekeeping gene glyceraldehyde-3-phosphate dehydrogenase (G3PDH). The G3PDH gene was detectable at 5-10 fewer PCR cycles in the control sample, demonstrating a reduction in abundance of between 2⁵ and 2¹⁰ fold in the subtracted sample.

### (3) Cloning, screening and sequencing of SSH-PCR products.

The PCR products resulting from the SSH-PCR were ligated into the pGEM-T easy vector (Promega Laboratories, Madison, WI, USA) and the ligations transformed into *Escherichia coli* TOP 10 competent cells (Invitrogen). A large number of transformants were obtained and 1,440 were replica plated onto 155 mm LB agar plates.

To screen the replica plated clones for increased expression during OTA production a portion of each colony was picked and emulsified in 100µl of sterile water, 2µl of each colony suspension was then used as a template in a PCR reaction using the PCR primers used to amplify the final SSH-PCR products. The PCR products obtained from this procedure were spotted onto duplicate nitrocellulose membranes (2.5µl per spot) and the DNA was fixed to the membranes by ultraviolet (UV) transillumination. The prepared membranes were then used in a hybridisation experiment to identify up-regulated clones. One membrane from each pair was probed with ³²P-ATP labelled cDNA from a permissive *A. ochraceus* culture and the other with ³²P-ATP labelled cDNA from a restrictive culture. Up-regulated clones were taken to be those for which the PCR product hybridised to the permissive probe but not to the restrictive probe. Clones that hybridised to both probes were disregarded. A total of 500 were screened of which 230 (46%) were upregulated (i.e. hybridised to permissive cDNA but not to restrictive cDNA). 192 up-regulated clones were selected for DNA sequencing. The nucleotide sequence of the cloned DNAs was determined and the deduced amino acid sequence of each sequence was compared to the protein sequence databases using the BLAST-X algorithm. The BLAST-X search translated the sequences in all 6 possible reading frames and compared them to the protein sequence databases (Table 1). 83 (55%) of the sequences were identifiable following the BLAST-X search. The largest number of identifiable clones encoded proteins with a role in cell metabolism, in addition about 30% of the sequences were classed as miscellaneous in that they could not be assigned to any of the several groupings. A number of clones with a potential role in ochratoxin biosynthesis were selected for further study. The nucleotide sequences of the selected clones are given in appendix 1.

### (4) Further studies on the cloned sequences

The presence of the cloned sequences in the genomic DNA of *A. ochraceus* and *A. carbonarius* 23804 was demonstrated by PCR using primers designed to the cloned sequences.

Reverse transcription-PCR was used to measure the expression of each of the cloned genes during OTA production.

**Table 1**

| BLASTX hit | size of clone (bp) |
|---|---|
| Averantin oxidoreductase | 630 |
| HC toxin synthase | 371 |
| Regulatory gene | 666 |
| 3-oxoacyl synthase | 846 |
| Trichodiene oxygenase | 572 |
| Acyl CoA dehydrogenase | 670 |
| Polyketide synthase | 408 |

The most significant appears to be a gene encoding a protein sequence similar to a number of polyketide synthase (PKS) proteins. As the ochratoxin A molecule contains a polyketide structure a polyketide synthase gene is essential for its biosynthesis. The presence of all of the genes in the *A. ochraceus* genome was confirmed by PCR with primers designed to each of the cloned sequences. Most of the PCR primer pairs also amplified products of the correct size from the OTA producing fungus *Aspergillus carbonarius* 23804.

To confirm that the genes were up-regulated during OTA biosynthesis the RNA was isolated at a number of time points [4, 5, 6, 7, 8, and 10 days] from *A. ochraceus* cultures growing on permissive and restrictive medium and used in a RT-PCR experiment to estimate the expression level for each clone.

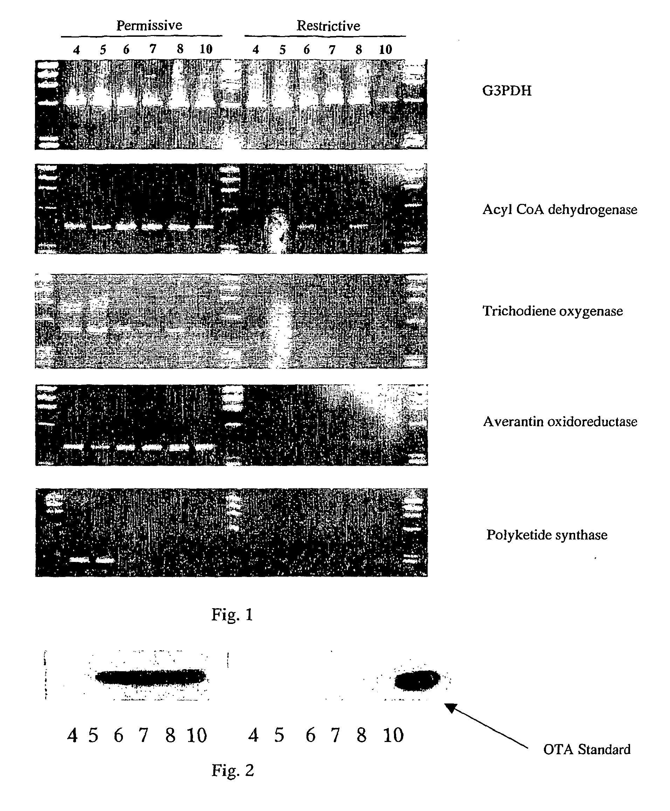

Reverse transcription-PCR (RT-PCR) studies of the expression of 4 of the selected clones under growth conditions permissive and restrictive for OTA production was carried out. Total RNA was extracted from *Aspergillus ochraceus* mycelium at different time-points during growth. cDNA was prepared from each RNA sample and used as a template in a PCR reaction with primers specific to each of the selected genes. Primers to the glyceraldehyde 3- phosphate dehydrogenase gene that is constitutively expressed were used in control reactions. OTA production was measured at each time point [4, 5, 6, 7, 8, and 10 days] by thin-layer chromatography to confirm that the growth conditions were permissive or restrictive as appropriate (Fig. 2).

The reverse transcription step produces DNA complementary to all RNA molecules in a particular sample thus the presence of a product in the subsequent PCR reaction is evidence that the RNA transcript for a specific gene was present at a particular time point.

The RT-PCR assay detects the level of transcription (production of mRNA) for a specific gene. RNA was isolated from fungal mycelia under permissive (OTA being produced) and restrictive (OTA not being produced) conditions. The RNA transcripts were reverse transcribed (complementary DNA (cDNA) molecules were synthesised) by the enzyme reverse transcriptase. The cDNA molecules were then detected by PCR. In the example no (or very low levels) transcripts from the OTA biosynthetic genes were detected when OTA was not being synthesised (Fig. 1).

As shown in Fig. 1 the glyceraldehyde-3-phosphate gene was expressed in all of the samples in both the permissive and restrictive cultures. The SSH-PCR clones were expressed strongly only in the permissive cultures. It was noticeable that the expression levels were highest at the earlier timepoints when it was most likely that OTA was being actively synthesised.

The use of an intron-spanning primer pair (AOKS-RT2) (Fig 1(f)) provides additional assurance that it is cDNA that has been detected rather than genomic DNA contamination. The intron is absent from cDNA having been spliced out of the RNA transcript. The genomic DNA product (lane 3) is therefore larger than that from cDNA (lane 4). The 2B11 primer products (that do not span an intron) from the genomic and cDNA respectively are in lanes 1 and 2 and are identical in size.

Primers used:
2B11-BF: 5'- ttctctactgcgcttctcacatccat-3'
2B11-BR: 5'- aacatcatagccataagaggtcaaca-3'
AOKSRT2-F: 5'- ctgacaccatcgaaaacctaaaaa -3'
AOKSRT2-R: 5'- tctaactcgcccttgacctg -3'

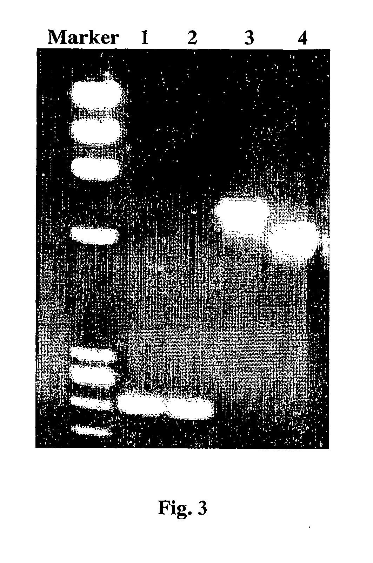

To confirm the involvement of the *pks* gene in OTA biosynthesis, a mutant of *A. ochraceus* [AO 118] was created which is incapable of producing ochratoxin A, through inactivation of the polyketide synthase (*pks*) gene. A hygromycin resistance gene cassette was inserted into the *pks* gene on the chromosome of *A. ochraceus*, so that the *pks* sequence was interrupted. Disruption of the *pks* gene was confirmed by the acquisition of hygromycin resistance by *A. ochraceus*. The hygromycin resistant transformants were then screened by plating on coconut cream agar (CA), OTA is fat soluble and therefore will diffuse into the fat present in the coconut cream. As OTA is fluorescent under UV light, exposure of the plates to UV allows identification of OTA producing strains by the presence of a blue fluorescent 'halo' around the culture, identified by the arrow in Fig. 3.

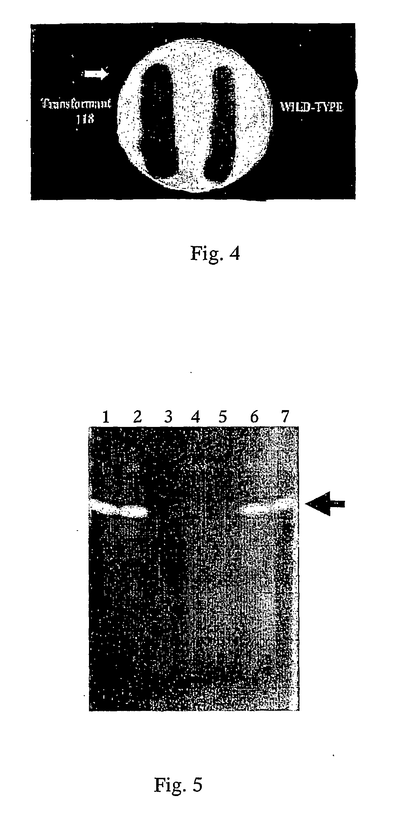

Transformants that appeared to be atoxigenic (OTA negative) after screening on CA were subjected to confirmatory testing by cutting out agar plugs from the medium and extracting the ochratoxin by treatment with acidified chloroform. The extracts [AO118, AO107 and AO53] were run on thin layer chromatography (TLC) plates that were visualised under UV light (Fig. 4). From these tests it is clear that the *pks* gene [7. SEQ ID No. 7 (Polyketide synthase (2179bp)] encodes a polyketide synthase gene which is essential for OTA production in *A. ochraceus*.

### Isolation of pks ketosynthase regions from Penicillium verrucosum and Aspergillus carbonarius.

Genomic DNA was isolated from *Aspergillus carbonarius* 23804 and *Penicillium verrucosum* OTA11 and used as a template in PCR reactions with degenerate primers designed from the deduced protein sequence of the ketosynthase region (KS) of the *A. ochraceus pks* gene. The PCR reactions produced products of the correct size from both fungi. The PCR product was purified by elution from an agarose gel and ligated into the pGEM-T easy vector. The ligation mixture was transformed into Top 10F chemically competent *Escherichia coli* cells (Invitrogen) and the transformed cells were plated on LB agar containing Ampicillin (100µg/ml), IPTG and X-GAL. A number of white colonies were selected and screened for the presence of the 2B11 PCR product by emulsifying a portion of the colony in 50µl of distilled water and using 2µl of the colony suspension as a template in a PCR reaction with the 2B11-B primer pair. A transformant containing the correct PCR product was selected and grown in LB broth; plasmid DNA was prepared from the LB culture using the Qiagen Spin Mini-Prep plasmid DNA isolation kit. The DNA sequence of the cloned PCR product was determined by Lark Technologies Inc., Saffron Walden, Essex, United Kingdom. The nucleotide sequence was compared to existing nucleotide and protein databases using the BLAST and BLAST-X programs on the NCBI website. Both sequences showed strong homology to the KS region of PKS proteins from a number of fungi, including some known to be implicated in the biosynthesis of polyketide mycotoxins.
Primers used
KS-DPA: 5'- GCIAAYGGITAYGCIMGIGG-3'
KS-DPB- 5'- GTICCIGTICCRTAIGCYTC -3'

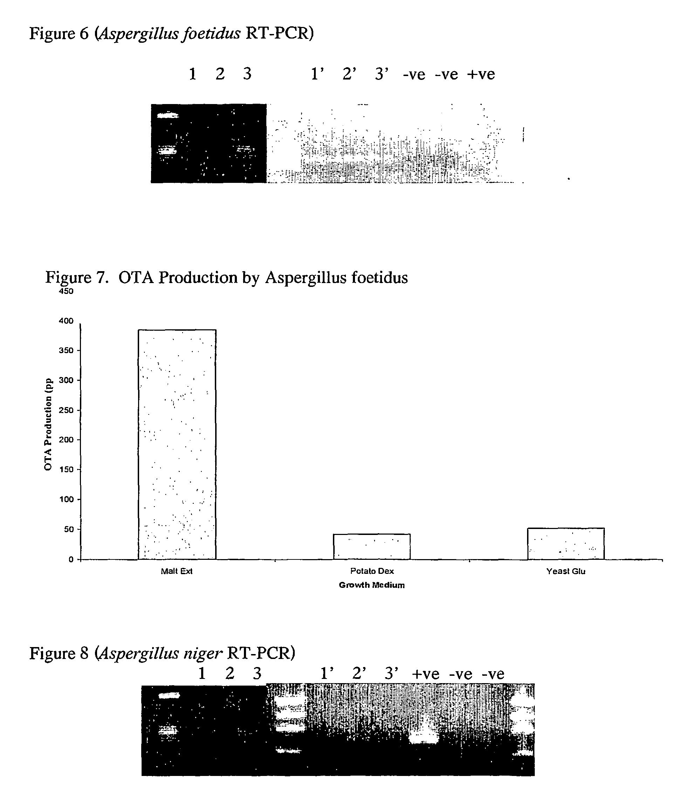

Referring to Fig. 8, no OTA was detected in the culture medium even though we have previously demonstrated OTA production by this strain. Measurement of the *pks* expression using the GAP primer pair in a RT-PCR assay showed that only a small amount of expression was occurring. This is in contrast to the higher level of *pks* expression observed in the *A. foetidus* strain that was producing OTA under these growth conditions (Figs. 6 and 7).

The PCR primer pairs used for the RT-PCR assay were designed from the *A. ochraceus* pks DNA sequence. The data in figures 6 and 8 demonstrates their application to the detection of OTA production in other *Aspergillus* species.

### Cloning of a pks homolog from Aspergillus niger.

Genomic DNA was isolated from *Aspergillus niger* 9029 and *Aspergillus foetius* 10254 and was used as a template in PCR reactions with two PCR primer pairs. The primer pair 2B 11-B produced a product from *A. niger* and the PKS4-GAP primer pair produced a product from *A. foetidus*. The products were the same size as those produced by these primers in *A. ochraceus*. The PCR products was purified by elution from an agarose gel and ligated into the pGEM-T easy vector. The ligation mixtures were transformed into Top 10f chemically competent *Escherichia coli* cells (Invitrogen) and the transformed cells were plated on LB agar containing Ampicillin (100µg/ml), IPTG and X-GAL. A number of white colonies were selected and screened for the presence of the 2B11 PCR product by emulsifying a portion of the colony in 50µl of distilled water and using 2µl of the colony suspension as a template in a PCR reaction with the 2B11-B primer pair. A transformant containing the correct PCR product was selected and grown in LB broth, plasmid DNA was prepared from the LB culture using the Qiagen Spin Mini-Prep plasmid DNA isolation kit. The DNA sequence of the cloned PCR product was determined by Lark Technologies Inc., Saffron Walden, Essex, United Kingdom. The nucleotide sequence was compared to existing nucleotide and protein databases using the BLAST and BLAST-X programs on the NCBI website.

Primers Used (all specific to the *pks* gene)
2B11-BF: 5'- ttctctactgcgcttctcacatccat-3'
2B11-BR: 5'- aacatcatagccataagaggtcaaca-3'
PKS4-GAPF: 5'- agccgtgttttcattctttc-3'
PKS4-GAPR: 5'- tgcggccatcttcgtgt-3'

### Primers used for Aspergillus

| Primer | Target gene. |
|---|---|
| AOB02-F: 5'- tatccgccgcctcgcaaactaat-3' | SEQ ID No. 6 |
| AOB02-R: 5'-cgaccgatcatgcgaccgtaaat -3' | SEQ ID No. 6 |
| | |
| AOB03-R: 5'-ctcggtgacatcaggggtatc-3' | SEQ ID No. 5 |
| AOB03-R: 5'-agcgtattcagtcactcattcaga-3' | SEQ ID No. 5 |
| | |
| AOE04-F: 5'-gctatgcgcggagaagtca-3' | SEQ ID No. 2 |
| AOE04-R: 5'-aaggctggggatcgtggagtg-3' | SEQ ID No. 2 |
| | |
| AOD07-F: 5'-agtttaccggccttgttga-3' | SEQ ID No. 4 |
| AOD07-R: 5'-ttattaccgtttgtcgctcttctc-3' | SEQ ID No. 4 |
| | |
| AOH11-F: 5'-agaacgggatgccaaaacagtgag-3' | SEQ ID No. 1 |
| AOH11 -R: 5'-aagaatgcgagggatgggataacc-3' | SEQ ID No. 1 |
| | |
| 2B11-BF: 5'- ttctctactgcgcttctcacatccat-3' | SEQ ID No. 7 |
| 2B11-BR: 5'- aacatcatagccataagaggtcaaca-3' | SEQ ID No. 7 |
| | |
| PKS4-GAPF: 5'- agccgtgttttcattctttc-3' | SEQ ID No. 7 |
| PKS4-GAPR: 5'- tgcggccatcttcgtgt-3' | SEQ ID No. 7 |
| | |
| KS-DPA: 5'- GCIAAYGGITAYGCIMGIGG-3' | SEQ ID No. 8 |
| KS-DPB- 5'- GTICCIGTICCRTAIGCYTC -3' | SEQ ID No. 8 |
| | |
| ACKS-1F: 5'-tgggtatgcgcggggtgagggtat-3' | SEQ ID No. 8 |
| ACKS-1R: 5'-ccgtaggcttcgaaaaactgacac-3' | SEQ ID No. 8 |

### Primers used for Penicillium

| Primer | Target gene |
|---|---|
| KS-DPA: 5'- GCIAAYGGITAYGCIMGIGG-3' | SEQ ID No. 9 |
| KS-DPB- 5'- GTICCIGTICCRTAIGCYTC -3' | SEQ ID No. 9 |
| | |
| PVKS-1F: 5'-tgcacgaccgggacaacatca-3' | SEQ ID No. 9 |
| PVKS-1R: 5'-ccgtaggcctccacaaaatctg-3' | SEQ ID No. 9 |

### Typical PCR assays utilising the invention

The methodology for the PCR assays depends on whether the assay is to detect the presence of fungi that are capable of producing OTA by detecting a biosynthetic gene (PCR) or to detect production of OTA by measuring expression of the biosynthetic gene (RT-PCR).

For the PCR assay a sample such as a grain sample will be ground to break up the grains and DNA extracted from the ground grains using a Qiagen DNeasy plant mini-kit (Qiagen GmBH), . The extracted DNA will be used as a template in a PCR reaction with one of the primer pairs listed above. Production of a PCR product of the correct size is taken as a positive result. The PCR assay can be performed on a real-time PCR apparatus such as a Light Cycler ™ (Roche Molecular Biochemicals) and this will provide results within 1 hour. The total time from receipt of samples to production of results can be as short as 2-3 hours.

For the RT-PCR assay, a sample such as a grain sample will be ground in liquid nitrogen and the RNA extracted using a commercially available extraction kit such as the RNeasy ^{™} (Qiagen GmBH). cDNA will then be prepared from the RNA by use of the reverse transcriptase enzyme. Once the cDNA has been prepared the procedure is identical to that for the PCR assay.

### Appendix 1

1. SEQ ID No. 1 (Averantin oxidoreductase (630bp))
2. SEQ ID No. 2 (HC Toxin synthase (371 bp))
3. SEQ ID No. 3 (Regulatory gene (666bp)).
4. SEQ ID No. 4 (3-oxoacyl synthase (846 bp))
5. SEQ ID No. 5 (Trichodiene oxygenase (572 bp))
6. SEQ ID No. 6 (Acyl CoA dehydrogenase (670 bp))
SEQ ID No. 7 (Polyketide synthase 2179 nucleotides):
SEQ ID No. 8 *Aspergillus carbonarius* ketosynthase (KS) region of the *pks* gene sequence (314 nucleotides):
SEQ ID No. 9 *P. verrucosum* ketosynthase (KS) region of the pks gene sequence (311 nucleotides):

### References

Burdaspal, P.A. and T.M. Legarda (1998). Ochratoxin A in roasted and soluble coffees marketed in Spain. Alimentaria, 35:103-109.
Burdaspal, P.A. and T.M. Legarda. (1999) Ochratoxin A in wines and grape products originated from Spain and other European countries. Alimentaria, 36:107-113.
Dietrich, D.R., C. Schlatter, I. Studer-Rohr and J. Schlatter. (1995) The Occurrence of Ochratoxin A in Coffee. Food and Chemical Toxicology, Volume 33, Issue 5, May 1995, Pages 341-355.
Edwards, S.G., J. O'Callaghan and A.D.W. Dobson (2002) PCR based detection and quantification of mycotoxigenic fungi. Mycol. Res. 106:1005-1025.
Geisen, R. (1998) PCR methods for the detection of mycotoxin producing fungi. In Applications of PCR in mycology (P.D. Bridge, D.K. Arora, C.A. Reddy, and R.P. Elander, eds): 243-246. CAB International, Wallingford.
Jorgenson, K. (1998) Survey of pork, poultry, coffee, beer and pulses for ochratoxin A. Food Addit Contam. 16:75-78.
Kuiper-Goodman, T. (1996) Risk assessment of ochratoxin A: an update. Food Addit. Contam. 13 (suppl): S53-S57.
Pittet, A. (1998) Natural occurrence of mycotoxins in food and feeds- an updated review. Rev. Med. Vet 149:479-492.
Scientific Committee for Food (1996). Opinion on aflatoxin, ochratoxin A and patulin, expressed on 23rd September 1994. Reports of the Scientific Committee for Food. 35th series. Luxembourg
Visconti, A., M. Pascale and G. Centonze. (1999) Determination of ochratoxin A in wine by means of immunoaffinity column clean-up and high-performance liquid chromatography, Journal of Chromatography A, Volume 864, Issue 1, 9 December 1999, Pages 89-101.
Visconti, A., M. Pascale and G. Centonze (2000) Determination of ochratoxin A in domestic and imported beers in Italy by immunoaffinity clean-up and liquid chromatography. Journal of Chromatography A, 888:321-326
Wolff, J. H. Bresch, C. Cholmakov-Bodechtel, G. Engel, M. Garais, P. Majerus, H. Rosner, R. Scheuer. (2000) Ochratoxin A: contamination of foods and consumer exposure. Arch. Lebensmittelhyg. 51:81-128.
Zimmerli, B and R. Dick. (1996) Ochratoxin A in table wine and grape juice: occurrence and risk assessment. Food Addit. Contam. 13:655-668.

## Claims

1. Use of a gene comprising the nucleotide sequence of SEQ ID No.1 which is expressed by fungal cells producing ochratoxin A in the detection and/or identification of ochratoxigenic fungi.

2. Use as claimed in claim 1 wherein the nucleotide sequence of SEQ ID No.1 is upregulated during ochratoxin A biosynthesis.

3. Use of a protein having an amino acid sequence encoded by the nucleotide sequence of SEQ ID No.1 in the detection and/or identification of an ochratoxin A producing fungi.

4. Use as claimed in claim 3 in the detection and/or identification of fungi that express ochratoxin A biosynethetic genes.

5. Use of a gene comprising the nucleotide sequence of SEQ ID No.1 in the construction of an atoxigenic strain of an ochratoxigenic fungus.

6. Use as claimed in any preceding claim wherein the ochratoxigenic fungus is of the genus *Aspergillus*.

7. Use as claimed in claim 6 wherein the fungus is of the species selected from any one of *carbonarius, niger, alliaceus* and *foetidus*.

8. Use as claimed in any of claims 1 to 5 wherein the ochratoxigenic fungus is of the genus *Penicillium.*

9. Use as claimed in claim 8 wherein the fungus is of the species *verrucosum*.

10. An isolated poly-nucleotide having the sequence of SEQ ID No 1.

11. An oligonucleotide primer for detection and/or identification of ochratoxigenic fungi selected from one or both of:
| | | |
|---|---|---|
| AOH11-F: | 5'-agaaegggatgccaaaacaetgas-3' | SEQ ID No. 18 |
| AOH11-R: | 5'-aagaatgcgagggatgggataacc-3' | SEQ ID No. 19 |

12. An assay for the detection of ochratoxin A producing fungi comprising the steps of:
(i) obtaining a sample;
(ii) extracting DNA
(iii) using the extracted DNA as a template to amplify DNA sequence or sequences to which the AOH11-F and AOH11-R primers are specific; and
(iv) analysing the amplified sequences.

13. An assay for the detection and identification of ochratoxin A producing genes comprising the steps of:
(i) obtaining a sample;
(ii) extracting RNA
(iii) synthesising complementary DNA (DNA)
(iv) using the synthesised cDNA as a template to amplify cDNA sequence or sequences to which the AOH11-F and AOH11-R primers are specific; and
(v) analysing the amplified sequences.

14. Use as claimed in claim 1, to identify or isolate a nucleotide sequence, gene, peptide or polypeptide involved in ochratoxin A biosynthesis.

## Patentansprüche

1. Verwendung eines Gens, das die Nukleotidsequenz von SEQ ID NO: 1 umfasst, welches von Ochratoxin A produzierenden Pilzzellen exprimiert wird, beim Nachweis und/oder bei der Identifikation von ochratoxigenen Pilzen.

2. Verwendung nach Anspruch 1, worin die Nukleotidsequenz von SEQ ID NO: 1 während der Biosynthese von Ochratoxin A hochreguliert wird.

3. Verwendung eines Proteins mit einer Aminosäuresequenz, das durch die Nukleotidsequenz von SEQ ID NO: 1 kodiert ist, beim Nachweis und/oder bei der Identifikation von einem Ochratoxin A produzierenden Pilz.

4. Verwendung nach Anspruch 3 beim Nachweis und/oder bei der Identifikation von Pilzen, die Ochratoxin A biosynthetische Gene exprimieren.

5. Verwendung eines Gens, das die Nukleotidsequenz von SEQ ID NO: 1 umfasst, bei der Konstruktion eines atoxigenen Stamms von einem ochratoxigenen Pilz.

6. Verwendung nach einem der vorangehenden Ansprüche, worin der ochratoxigene Pilz aus dem Genus *Aspergillus* ist.

7. Verwendung nach Anspruch 6, worin der Pilz von der Species ist, die aus jedweder einen von *A. carbonarius, A. niger, A. alliaceus* und *A. foetidus* ausgewählt ist.

8. Verwendung nach einem der Ansprüche 1 bis 5, worin der ochratoxigene Pilz aus dem Genus *Penicillium* ist.

9. Verwendung nach Anspruch 8, worin der Pilz von der Species *P. verrucosum* ist.

10. Isoliertes Polynukleotid mit der Sequenz von SEQ ID NO: 1.

11. Oligonukleotid-Primer zum Nachweis und/oder zur Identifikation von ochratoxigenen Pilzen, der aus einem oder beidem von Folgendem ausgewählt ist:
| | | |
|---|---|---|
| AOH11-F: | 5'-agaacgggatgccaaaacagtgag-3' | SEQ ID NO: 18 |
| AOH11-R: | 5'-aagaatgcgagggatgggataacc-3' | SEQ ID NO: 19 |

12. Assay zum Nachweis von Ochratoxin A produzierenden Pilzen, der die folgenden Schritte umfasst:
(i) Gewinnen einer Probe;
(ii) Extrahieren von DNA;
(iii) Verwenden der extrahierten DNA als ein Template zum Amplifizieren der DNA-Sequenz oder -Sequenzen, für welche die AOH11-F- und AOH11-R-Primer spezifisch sind; und
(iv) Analysieren der amplifizierten Sequenzen.

13. Assay zum Nachweis und zur Identifikation von Ochratoxin A produzierenden Genen, der die folgenden Schritte umfasst:
(i) Gewinnen einer Probe;
(ii) Extrahieren von RNA;
(iii) Synthetisieren von komplementärer DNA (cDNA);
(iv) Verwenden der synthetisierten cDNA als ein Template zum Amplifizieren der cDNA-Sequenz oder -Sequenzen, für welche die AOH11-F- und AOH11-R-Primer spezifisch sind; und
(v) Analysieren der amplifizierten Sequenzen.

14. Verwendung nach Anspruch 1, zum Identifizieren oder Isolieren einer Nukleotidsequenz, eines Gens, eines Peptids oder Polypeptids, die an der Biosynthese von Ochratoxin A beteiligt sind.

## Revendications

1. Utilisation d'un gène comprenant la séquence nucléotidique de SEQ ID No 1 qui est exprimé par des cellules fongiques produisant l'ochratoxine A dans la détection et/ou l'identification de champignons ochratoxinogènes.

2. Utilisation selon la revendication 1, dans laquelle la séquence nucléotidique de SEQ ID No 1 est régulée à la hausse durant la biosynthèse de l'ochratoxine A.

3. Utilisation d'une protéine ayant une séquence d'acides aminés codée par la séquence nucléotidique de SEQ ID No 1 dans la détection et/ou l'identification de champignons produisant l'ochratoxine A.

4. Utilisation selon la revendication 3 dans la détection et/ou l'identification de champignons qui expriment des gènes de biosynthèse de l'ochratoxine A.

5. Utilisation d'un gène comprenant la séquence nucléotidique de SEQ ID No 1 dans la construction d'une souche atoxinogène d'un champignon ochratoxinogène.

6. Utilisation selon l'une quelconque des revendications précédentes, dans laquelle le champignon ochratoxinogène est du genre *Aspergillus.*

7. Utilisation selon la revendication 6, dans laquelle le champignon est de l'espèce choisie parmi l'un quelconque de *carbonarius, niger, alliaceus et foetidus.*

8. Utilisation selon l'une quelconque des revendications 1 à 5, dans laquelle le champignon ochratoxinogène est du genre *Penicillium.*

9. Utilisation selon la revendication 8, dans laquelle le champignon est de l'espèce *verrucosum.*

10. Polynucléotide isolé ayant la séquence de SEQ ID No 1.

11. Amorce oligonucléotidique pour la détection et/ou l'identification de champignons ochratoxinogènes choisis dans l'un ou les deux parmi :
| | | |
|---|---|---|
| AOH11-F : | 5'-agaacgggatgccaaaacagtgag-3' | SEQ ID No 18 |
| AOH11-R : | 5'-aagaatgcgagggatgggataacc-3' | SEQ ID No 19 |

12. Dosage pour la détection de champignons produisant l'ochratoxine A, comprenant les étapes consistant à :
(i) obtenir un échantillon ;
(ii) extraire l'ADN ;
(iii) utiliser l'ADN extrait comme matrice pour amplifier la séquence ou les séquences d'ADN de laquelle ou desquelles les amorces AOH11-F et AOH11-R sont spécifiques ; et
(iv) analyser les séquences amplifiées.

13. Dosage pour la détection et/ou l'identification de gènes produisant l'ochratoxine A, comprenant les étapes consistant à :
(i) obtenir un échantillon ;
(ii) extraire l'ARN ;
(iii) synthétiser l'ADN complémentaire (ADNc) ;
(iv) utiliser l'ADNc synthétisé comme matrice pour amplifier la séquence d'ADNc ou les séquences d'ADNc de laquelle ou desquelles les amorces AOH11-F et AOH11-R sont spécifiques ; et
(v) analyser les séquences amplifiées.

14. Utilisation selon la revendication 1 pour identifier ou isoler une séquence nucléotidique, un gène, un peptide ou un polypeptide impliqué dans la biosynthèse de l'ochratoxine A.


### SEQUENCE LISTING

<110> University College Cork - Notional University of Ireland, Cork
<120> Detection of ochratoxin A producing fungi
<130> NATI25
<160> 29
<170> PatentIn version 3.2
<210> 1
   <211> 630
   <212> DNA
   <213> Aspergillus ochraceus
<220>
   <221> misc_feature
   <222> (34)..(34)
   <223> n is a, c, g, or t
<220>
   <221> misc_reature
   <222> (74)..(74)
   <223> n is a, c, g, or t
<220>
   <221> misc_feature
   <222> (76)..(76)
   <223> n is a, c, g, or t
<220>
   <221> misc_feature
   <222> (83)..(83)
   <223> n is a, c, g, or t
<220>
   <221> misc_feature
   <222> (373)..(373)
   <223> n is a, c, g, or t
<220>
   <221> misc_feature
   <222> (382)..(382)
   <223> n is a, c, g, or t
<400> 1
<21 0> 2
   <211> 371
   <212> DNA
   <213> Aspergillus ochraceus
<220>
   <221> misc_feature
   <222> (39)..(39)
   <223> n is a, c, g, or t
<220>
   <221> misc_feature
   <222> (44)..(44)
   <223> n is a, c, g, or t
<220>
   <221> misc_feature
   <222> (76)..(76)
   <223> n is a, c, g, or t
<220>
   <221> misc_feature
   <222> (79)..(79)
   <223> n is a, c, g, or t
<400> 2
<210> 3
   <211> 666
   <212> DNA
   <213> Aspergillus ochraceus
<220>
   <221> misc_feature
   <222> (38)..(38)
   <223> n is a, c, g, or t
<220>
   <221> misc_feature
   <222> (639)..(639)
   <223> n is a, c, g, or t
<220>
   <221> misc_feature
   <222> (642)..(642)
   <223> n is a, c, g, or t
<400> 3
<210> 4
   <211> 844
   <212> DNA
   <213> Aspergillus ochraceus
<220>
   <221> misc_feature
   <222> (37)..(37)
   <223> n is a, c, g, or t
<220>
   <221> misc_feature
   <222> (535)..(535)
   <223> n is a, c, g, or t
<220>
   <221> misc_feature
   <222> (595)..(595)
   <223> n is a, c, g, or t
<220>
   <221> misc_feature
   <222> (602)..(602)
   <223> n is a, c, g, or t
<220>
   <221> misc_feature
   <222> (631)..(631)
   <223> n is a, c, g, or t
<220>
   <221> misc_feature
   <222> (637)..(637)
   <223> n is a, c, g, or t
<220>
   <221> misc_feature
   <222> (718)..(718)
   <223> n is a, c, g, or t
<220>
   <221> misc_feature
   <222> (728).. (728)
   <223> n is a, c, g, or t
<220>
   <221> misc_feature
   <222> (731)..(731)
   <223> n is a, c, g, or t
<220>
   <221> misc_feature
   <222> (745)..(745)
   <223> n is a, c, g, or t
<220>
   <221> misc_feature
   <222> (748)..(748)
   <223> n is a, c, g, or t
<220>
   <221> misc_feature
   <222> (754)..(754)
   <223> n is a, c, g, or t
<220>
   <221> misc_feature
   <222> (756)..(756)
   <223> n is a, c, g, or t
<220>
   <221> misc_feature
   <222> (779)..(779)
   <223> n is a, c, g, or t
<220>
   <221> misc_feature
   <222> (794)..(794)
   <223> n is a, c, g, or t
<220>
   <221> misc_feature
   <222> (797)..(797)
   <223> n is a, c, g, or t
<220>
   <221> misc_feature
   <222> (810)..(811)
   <223> n is a, c, g, or t
<220>
   <221> misc_feature
   <222> (814)..(814)
   <223> n is a, c, g, or t
<400> 4
<210> 5
   <211> 582
   <212> DNA
   <213> Aspergillus ochraceus
<220>
   <221> misc_feature
   <222> (37)..(37)
   <223> n is a, c, g, or t
<220>
   <221> misc_feature
   <222> (40)..(41)
   <223> n is a, c, g, or t
<220>
   <221> misc_feature
   <222> (73)..(73)
   <223> n is a, c, g, or t
<400> 5
<210> 6
   <211> 670
   <212> DNA
   <213> Aspergillus ochraceus
<400> 6
<210> 7
   <211> 2179
   <212> DNA
   <213> Aspergillus ochraceus
<400> 7
<210> 8
   <211 > 314
   <212> DNA
   <213> Penicillium verrucosum
<220>
   <221> misc_feature
   <222> (265)..(265)
   <223> n is a, c, g, or t
<400> 8
<210> 9
   <211> 311
   <212> DNA
   <213> Penicillium verrucosum
<400> 9
<210> 10
   <211> 23
   <212> DNA
   <213> artificial seqeunce
<220>
   <223> primer
<400> 10
   tatccgccgc ctcgcaaact aat
   23
<210> 11
   <211> 23
   <212> DNA
   <213> artificial sequence
<220>
   <223> primer
<400> 11
   cgaccgatca tgcgaccgta aat
   23
<210> 12
   <211> 21
   <212> DNA
   <213> artificial sequence
<220>
   <223> primer
<400> 12
   ctcggtgaca tcaggggtat c
   21
<210> 13
   <211> 24
   <212> DNA
   <213> artificial sequence
<220>
   <223> primer
<400> 13
   agcgtattca gtcactcatt caga
   24
<210> 14
   <211> 19
   <212> DNA
   <213> artificial sequence
<220>
   <223> primer
<400> 14
   gctatgcgcg gagaagtca
   19
<210> 15
   <211> 21
   <212> DNA
   <213> artificial sequence
<220>
   <223> primer
<400> 15
   aaggctgggg atcgtggagt g
   21
<210> 16
   <211> 19
   <212> DNA
   <213> artificial sequence
<220>
   <223> primer
<400> 16
   agtttaccgg ccttgttga
   19
<210> 17
   <211> 24
   <212> DNA
   <213> artificial sequence
<220>
   <223> primer
<400> 17
   ttattaccgt ttgtcgctct tctc
   24
<210> 18
   <211> 24
   <212> DNA
   <213> artificial sequence
<220>
   <223> primer
<400> 18
   agaacgggat gccaaaacag tgag 24
<210> 19
   <211> 24
   <212> DNA
   <213> artificial sequence
<220>
   <223> primer
<400> 19
   aagaatgcga gggatgggat aacc 24
<210> 20
   <211> 26
   <212> DNA
   <213> artificial sequence
<220>
   <223> primer
<400> 20
   ttctctactg cgcttctcac atccat 26
<210> 21
   <211> 26
   <212> DNA
   <213> artificial sequence
<220>
   <223> primer
<400> 21
   aacatcatag ccataagagg tcaaca 26
<210> 22
   <211> 20
   <212> DNA
   <213> artificial sequence
<220>
   <223> pimer
<400> 22
   agccgtgttt tcattctttc
   20
<210> 23
   <211> 17
   <212> DNA
   <213> artificial sequence
<220>
   <223> primer
<400> 23
   tgcggccatc ttcgtgt
   17
<210> 24
   <211> 20
   <212> DNA
   <213> artificial sequence
<220>
   <223> primer
<220>
   <221> misc_feature
   <222> (3)..(3)
   <223> n is i
<220>
   <221> misc_feature
   <222> (9)..(9)
   <223> n is i
<220>
   <221> misc_feature
   <222> (15)..(15)
   <223> n is i
<220>
   <221> misc_feature
   <222> (18)..(18)
   <223> n is i
<400> 24
   gcnaayggnt aygcnmgngg
   20
<210> 25
   <211> 20
   <212> DNA
   <213> artificial sequence
<220>
   <223> primer
<220>
   <221> misc_feature
   <222> (3)..(3)
   <223> n is i
<220>
   <221> misc_feature
   <222> (6)..(6)
   <223> n is i
<220>
   <221> misc_feature
   <222> (9)..(9)
   <223> n is i
<220>
   <221> misc_feature
   <222> (15)..(15)
   <223> n is i
<400> 25
   gtnccngtnc crtangcytc
   20
<210> 26
   <211> 24
   <212> DNA
   <213> artificial sequence
<220>
   <223> primer
<400> 26
   tgggtatgcg cggggtgagg gtat
   24
<210> 27
   <211> 24
   <212> DNA
   <213> artificial sequence
<220>
   <223> primer
<400> 27
   ccgtaggctt cgaaaaactg acac
   24
<210> 28
   <211> 21
   <212> DNA
   <213> artificial sequence
<220>
   <223> primer
<400> 28
   tgcacgaccg ggacaacatc a
   21
<210> 29
   <211> 22
   <212> DNA
   <213> artificial sequence
<220>
   <223> primer
<400> 29
   ccgtaggcct ccacaaaatc tg
   22